# EUROPEAN PATENT SPECIFICATION

(11) **EP 2 918 245 B1**
(45) Date of publication and mention of the grant of the patent: **03.05.2017**
(21) Application number: 14160061.9
(22) Date of filing: 14.03.2014
(51) Int. Cl.: A61F 2/24

(54) **Heart valve comprising a crown piece interconnected to leaflets, a top cuff and a bottom cuff; and a medical implant**
Herzklappe mit einem Stück Krone, das mit Blättchen, einer oberen Manschette und einer unteren Manschette verbunden ist; und medizinisches Implantat
Valve cardiaque comprenant une pièce de sommet interconnectée avec des feuillets, revers supérieur et manchon inférieur et implant médical

(43) Date of publication of application: 16.09.2015
(73) Proprietor: Venus MedTech (HangZhou), Inc., Hangzhou, Zhejiang (CN)
(72) Inventor: Lim, Hou-Sen, 455234 Singapore (SG); Götz, Wolfgang, 93051 Regensburg (DE)
(74) Representative: Bobbert & Partner Patentanwälte PartmbB

(56) References cited:
- WO-A1-2014/004806
- WO-A2-2008/029296
- CA-A1- 2 412 063
- US-A1- 2004 210 305
- US-A1- 2006 287 717

## Description

The present invention relates to a heart valve according to claim 1 and to a medical implant (short hereinafter: implant) according to claim 7.
From WO 2009/109348 A1, implants comprising a frame and a heart valve attached to the frame are known.
One object of the present invention is to provide another heart valve and another medical implant comprising such a heart valve.
This object may be solved by a heart valve having the features of claim 1. Insofar as the terms "invention" and/ or "embodiment" are used in the following, and/or features are presented as being optional, this should be interpreted in such a way that the only protection sought is that of the invention as claimed. According to the present invention, a heart valve is suggested which comprises at least two heart valve leaflets. It also comprises at least one crown piece (also referred to as 'triangle' hereinafter) interconnected to the leaflets. The crown piece is preferably intended to be interconnected, directly or indirectly, for example sewed, to a frame (also referred to as the support or the body of the implant or a stent by way of example hereinafter).
The heart valve further comprises a top cuff and a bottom cuff.

The crown piece, the top cuff and the bottom cuff are each rings or ring-shaped, and both the top cuff and the bottom cuff are interconnected with the crown piece.

Also, according to the present invention, a medical implant comprising a heart valve according to the present invention is suggested. The implant has a frame or support or stent that is foldable and/or unfoldable.

The frame comprises at least or exactly one first guiding structure for guiding or comprising at least one tension thread for folding and/or unfolding the frame around or along the frame, for example at an outside or an circumference thereof.

The frame comprises at least or exactly one second guiding structure different from the first guiding structure also for guiding or comprising at least one tension thread for folding and/or unfolding the implant around or along the frame, for example at an outside or an circumference thereof.

The frame further comprises at least two, preferably three, posts. The posts are arranged between the first and the second guiding structure such that they interconnect the first and the second guiding structure with each other and/or maintain the distance between them.

In the following, the use of the expression "may be" or "may have", and so on, is to be understood synonymously with "in exemplary embodiments is" or "in exemplary embodiments has", respectively, and so on, and is intended to illustrate exemplary embodiments according to the present invention.

Exemplary embodiments according to the present invention are each also subject of dependent claims.

Exemplary embodiments according to the present invention may comprise one or more of the features named hereinafter.

In certain exemplary embodiments according to the present invention, the implant may be folded or unfolded upon implantation by using one or several tension threads or filaments wound around the implant.

In some exemplary embodiments according to the present invention, altering the shape of the implant means reducing or increasing a diameter, particularly an external diameter, of the implant. Such an alteration may or may not involve an alteration of the implant's length or any other kind of alteration.

In certain exemplary embodiments according to the present invention, folding the implant means reducing the diameter of the implant. Folding also covers "re-folding" of an once expanded implant.

In some exemplary embodiments according to the present invention, unfolding should be understood as increasing the diameter of the implant, or as expanding.

In certain exemplary embodiments according to the present invention, the diameter of the implant is arranged in a plane perpendicular to a main flow direction of the implant in case fluids flow through the implant after its implantation.

In some exemplary embodiments according to the present invention, at least one of the top cuff and the bottom cuff is formed from a stripe (or strap) or comprises a stripe (or strap, e.g. a thin band that is longer than broad). The stripe is (in its flat state) curved along its length, preferably or at least in a plane of its width.

In certain exemplary embodiments according to the present invention, the top cuff has a width that is smaller than a width of the bottom cuff.

In certain exemplary embodiments according to the present invention, the 'width' refers to an average width of the stripe.

In some exemplary embodiments according to the present invention, the top cuff and the bottom cuff are equally long (or almost equally long).

In particular exemplary embodiments according to the present invention, the top cuff is arranged closer to the leaflets than the bottom cuff.

In some exemplary embodiments according to the present invention, all leaflets are sewed to the crown piece by means of one or exactly one suture or stitch.

In particular exemplary embodiments according to the present invention, a suture is a filament or a thread or yarn. In these embodiments, 'one suture' means one filament (or thread or yarn) used for sewing two pieces together. In these embodiments, no second filament is used and, in consequence, only one knot is required.

In some exemplary embodiments according to the present invention, both the top cuff and the bottom cuff are sewed to the crown piece by means of one or exactly one suture.

In particular exemplary embodiments according to the present invention, the crown piece is sewed to the posts, preferably using (or via or through) through holes or eyelets of the posts, preferably through at least three or four, preferably consecutive through holes, preferably by means of tabs being small extensions of the crown piece or by leaving out the tabs, preferably by means of one or exactly one suture per post.

In some exemplary embodiments according to the present invention, the suture for interconnecting the crown piece to one post was started from an outer side of that post to an inner side of the post. Preferably, the single knot that interconnects both ends of the suture is arranged on an outside of the post.

In particular exemplary embodiments according to the present invention, the posts are arranged inside a circle or an area circumscribed by the crown piece.

In some exemplary embodiments according to the present invention, the posts are arranged outside the circle or the area circumscribed by at least one of the top cuff and the bottom cuff.

In particular exemplary embodiments according to the present invention, at least one of the first and the second guiding structure comprises or consists of bars (can be struts instead) that are interconnected to each other so as to form a zig-zag pattern or an undulating or meandering pattern. Neighbouring or adjacent or contacting bars are provided for moving relative to each other or for changing a distance or an angle between them (or between sections thereof, respectively) upon folding or unfolding of the implant or frame. The bars are preferably arranged outside the circle or the area circumscribed by at least one of the top cuff and the bottom cuff. In preferred embodiments according to the present invention the bars are covered on their inner side (being the side towards the inner space of the frame or implant) at least in part(s) by at least one of the top cuff and the bottom cuff.

In some exemplary embodiments according to the present invention, the leaflets have a first tab and a second tab arranged at opposite ends of the respective leaflet. The tabs are sewed onto the post of the frame.

In certain exemplary embodiments according to the present invention, the tabs extend from the body of the leaflet.

In particular exemplary embodiments according to the present invention, tabs of two adjacent leaflets are sewed to one post in an overlapping manner.

In certain exemplary embodiments according to the present invention, the tab of a first leaflet is or was sewed onto a post first, and wherein the tab of a second leaflet was sewed onto both the tab of a first leaflet and the post the tab of the first leaflet had been sewed to, all in one running stitch or with one suture.

In some exemplary embodiments according to the present invention, the medical implant comprises exactly three posts.

In certain exemplary embodiments according to the present invention, the heart valve comprises exactly three leaflets. In particular exemplary embodiments according to the present invention, the implant is a heart or cardiac valve assembly.

In certain exemplary embodiments according to the present invention, the crown piece may have up to three sections that are triangle in shape (at least once the crown piece's free ends are put together such that the crown piece forms a ring).

In particular exemplary embodiments according to the present invention, the frame (or support or body) of the implant is made of or comprises a metal or a shape memory material.

In certain exemplary embodiments according to the present invention, the top cuff and the bottom cuff are originally separate pieces, directly or indirectly interconnected with each other by sewing.

In some exemplary embodiments according to the present invention, the crown piece may have sections ending in a tip of a triangle section of the crown piece or in a tab extending from the tip of the triangle, the tab having a free end (before being fixed to, for example, the leaflets).

In certain exemplary embodiments according to the present invention, the crown piece is interposed between the top cuff and the bottom cuff.

In certain exemplary embodiments according to the present invention, at least one of the top cuff and the bottom cuff is made from porcine pericardium or is a fabric.
In some exemplary embodiments according to the present invention, the leaflets are interconnected with, preferably glued or sewed to, the crown piece.
In some exemplary embodiments of the apparatus according to the present invention, the at least one tension thread is a thread. The thread may be a surgical suture thread or similar to it. The thread may have the shape of a rope, a filament or a cord. The thread may be designed as a chain having a plurality of engaging chain links.
In this specification, the term thread or tension thread may also define a plurality of threads or tension threads whenever a person skilled in the art recognizes the exchangeability of the terms.
In certain exemplary embodiments according to the present invention, the implant or its frame is permeable for fluids in its implanted state in its longitudinal direction. "Permeable" means that the fluid may flow through the implant, for example, through an inner lumen thereof.
In particular exemplary embodiments according to the present invention, the frame has features as described in WO 2011/063972 A1 or WO 2009/109348 A1 with respect to the implant.

In certain exemplary embodiments according to the present invention, the implant is configured to have or has tension applied to it by using at least one tension thread. The tension is preferably controlled by altering a length of the pulling device by which it extends out of the interior of the shaft or a catheter or sections thereof.

In some exemplary embodiments according to the present invention, at least one of the heart valve and the frame comprises exclusively, i.e. only, (one or more) materials that are MRI (short for: magnetic resonance imaging) compatible.

In certain exemplary embodiments according to the present invention, at least one of the heart valve and the frame comprises exclusively (one or more) materials that are not magnetic, ferromagnetic, or both.

In some exemplary embodiments according to the present invention, at least one of the heart valve and the frame does not comprise metal or any metal alloy.

In certain embodiments according to the present invention, eat least one of the posts has at least two openings through which tension threads are guided from an inside or inner space of the implant to an outside of the implant and back from the outside to the inside. The tension threads are guided to the outside through a first opening of a first one of the posts and back to the inside - or vice versa - through any second first opening of any second post, the first opening being different from the second opening, and the first post being different from the second post.

In particular exemplary embodiments according to the present invention, the crown piece is interconnected to the frame of a medical implant or a heart valve assembly.

In certain exemplary embodiments according to the present invention, both the top cuff and the bottom cuff are interconnected to bars of a guiding structure of the frame, preferably by sewing, preferably in direct contact to the bars.

Some or all advantages achievable by the heart valve according to the present invention may in certain exemplary embodiments of the present invention also be achieved by medical implant according to the present invention.

Some or all exemplary embodiments according to the present invention may provide for one, several or all of the advantages named above and/or hereafter.

According to the present invention, the top cuff and the bottom cuff may have different widths. If the top cuff and the bottom cuff is now everted to the outside face of the bars both at an upper end and an lower end of the bars by an equal distance (the equal distance is usually equal since it takes the same amount of material or overlap to secure the cuffs on the tips of both the upper parts and the lower parts of the bars), a suture interconnecting the two cuffs (plus the crown piece) will not be positioned in a middle line of the bars. That way, the suture will not be damaged by the bars in a folded state of the implant in which the middle of the bars will usually have to face the highest pressure. At the same token, the suture does not contribute to applying pressure on the leaflets starting about the height of the bars of the guiding structure as the suture will not contribute to narrowing the space about the middle line of the bars due to its position beyond the middle line.

Further, sewing parts by just one suture may contribute in avoiding knots which in turn require space and are prone to damaging neighboring structures such as leaflets.

If, as in particular embodiments according to the present invention, the inner side of the top cuff is interconnected to the outer side of the bottom cuff, the resulting geometrical shape will show a profile that extends with a middle portion thereof into the inner space it circumscribes. In a front cut the geometrical shape may be called concave. That shape may fit best to the also concave shape of the bent bars and the resulting concave shape of the guiding structure which is another advantage.

Providing at least one of the heart valve and the implant to be MRI compatible allows advantageously for controlling the location and orientation of the apparatus or the implant, or both, by MRI upon use of the apparatus or implantation of the implant. No heat, sparks or artefacts are generated during MRI because of the materials chosen for the apparatus or the implant.

In the following, examples of the present invention will be described with reference to the accompanying figures wherein similar or identical assemblies or elements are denoted by same reference numerals.
- **Fig. 1a**: shows three leaflets of a heart valve according to a first exemplary embodiment of the present invention;
- **Fig. 1b**: shows a crown piece of a heart valve according to the first exemplary embodiment of the present invention;
- **Fig. 1c**: shows a top cuff of a heart valve according to the first exemplary embodiment of the present invention;
- **Fig. 1d**: shows a bottom cuff of a heart valve according to the first exemplary embodiment of the present invention;
- **Fig. 1e**: shows three pledges of a heart valve according to the first exemplary embodiment of the present invention;
- **Fig. 2**: shows a medical implant according to the present invention in an expanded state which is expandable and can be reduced in its diameter by use of a means;
- **Fig. 3**: shows the medical implant of Fig. 2 in a non- or less expanded state;
- **Figs**. **4a-4c**: show how the heart valve of Figs. 1a to 1d or 1e is being fixed or secured to a frame according to Figs. 2 or 3;
- **Figs. 5a-5c**: show how tabs of adjacent leaflets of the heart valve according one embodiment of the present invention are commonly attached to one post of the frame;
- **Figs. 6a-6c**: show how a pledge is used for reinforcing the connection of the heart valve according to the frame of the present invention; and
- **Figs. 7a-7b**: show a top cuff and a bottom cuff sewed to the crown piece.

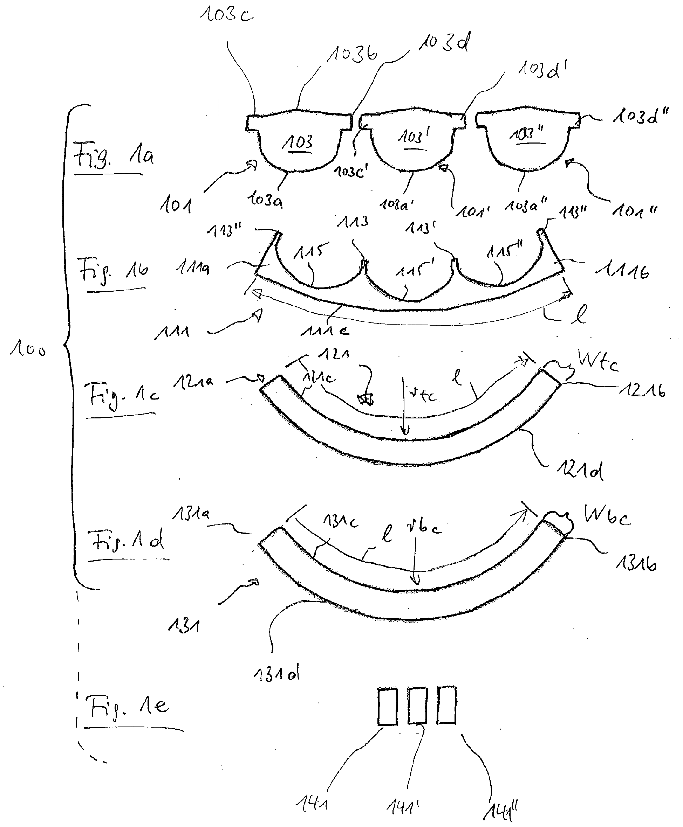

**Fig. 1a** through **Fig. 1e** show parts of a heart valve 100 according to a first exemplary embodiment of the present invention.

**Fig. 1a** shows three leaflets 101, 101' and 101" of the heart valve 100 of the first exemplary embodiment of the present invention. Instead of three leaflets 101, 101' and 101" the heart valve 100 according to the present invention may comprise any other number of leaflets, for example two. In the exemplary embodiment of Fig. 1a, all leaflets 101, 101' and 101" are identical. In other embodiments according to the present invention, at least two of them may, however, be different from each other.

In the exemplary embodiment of Fig. 1a, each leaflet 101, 101', 101" has a body 103, 103', 103", respectively, having a round or curved bottom section 103a, 103a', 103" and a rim section 103b, 103b', 103b" opposing the corresponding curved section 103a, 103a' or 103a". The rim section 103b, 103b', 103b" extends into opposing tabs 103c, 103c', 103" and 103d, 103d', 103d" which form the outmost portions to the opposing sides of the respective body 103, 103' or 103" (i. e. left and to the right of in the illustration of Fig. 1a).

**Fig. 1b** shows a crown piece 111 of the heart valve 100. In use, the crown piece 111 is formed to a ring by connecting together the free ends 111a and 111b of the stripe shown in Fig. 1b with each other.

The crown piece 111 optionally comprises small tabs 113, 113', 113" and round portions 115, 115' 115".

The round portions 115, 115' 115" are shaped such that their curved rims correspond to the curved sections 103a, 103a' or 103a" of the leaflets 101, 101', 101".

In Fig. 1b, the reference number 113" is used twice. In practice, both small (half-)tabs 113" will be contact each other so as to form one single small tab afterwards.

In certain embodiments according to the present invention, the leaflets 101, 101' and 101" and/or the crown piece 111 (and, if applicable, also the pledges (also referred to a pledgets, these terms being, hence, synonyms) 141, 141', 141" , see Fig. 1e) are cut (e. g. laser cut) from a (e. g. jib-fixed) bovine pericardium having a preferred thickness between 0.35 and 0.55 mm.

In some embodiments according to the present invention, the leaflets 101, 101' and 101" are all of identical or similar stiffness.

**Fig. 1c** shows a top cuff 121 of the heart valve 100. As can be seen from Fig. 1c, the top cuff 121, which is used in a ring-shaped form after having united the top cuff's ends 121a and 121b with each other, is formed from a flat stripe. The same applies to a bottom cuff 131 discussed below with reference to **Fig. 1d** showing a bottom cuff 131 with ends 131a, 131b of the heart valve 100 according to the first exemplary embodiment of the present invention.

The width of the top cuff 121 is denoted with wtc. The width of the bottom cuff 131 is denoted with wbc. In certain embodiments according to the present invention, wtc is smaller than wbc.

The length of the top cuff 121 is denoted with 1. The width of the bottom cuff 131 is denoted with 1 as well since in the exemplary embodiment shown in the figures the top cuff 121 and the bottom cuff 131 are of the same length, at least with respect to a first side 121c of the top cuff 121 and a second side 131c of the bottom cuff 131. "1" also denotes the length of the lower rim or side 111c of crown piece 111. All lengths denoted with 1 are identical in certain embodiments according to the present invention.

The reference numerals rtc and rbc of Fig. 1c and 1d denote the radius of the curvature of the top cuff 121 and the bottom cuff 131, respectively. The radius rtc and the radius rbc indicate that the stripes shown in Fig. 1c und 1d are not straight but bent within the drawing plane of Figs. 1c and 1d. rtc and rbc may be identical, without being limited hereto.

The inner side of top cuff 121 is denoted with 121c, the outer side with 121d.

The inner side of bottom cuff 131 is denoted with 131c, the outer side with 131d.

Because of the radius of top cuff 121 and bottom cuff 131, their inner sides 121c, 131c are shorter than their outer sides 121d, 131d, respectively.

In particular embodiments according to the present invention, the inner side 121c of top cuff 121 is interconnected with the outer side 131d of bottom cuff 131. This way, the resulting structure will be generally cylindrical with a middle (or about middle) portion that protrudes into the inner space formed by the resulting structure.

In certain embodiments according to the present invention, top cuff 121 and the bottom cuff 131 are cut (e. g. laser cut) from a (e. g. surface-tension) porcine pericardium having a preferred thickness between 0.15 and 0.25 mm.

The small tabs 113, 113', 113" may be used for a temporary stitch for temporarily securing the crown piece 111 to the frame 1. Both the provided temporary stitch and the small tabs 113, 113', 113" may be cut off and disposed later on.

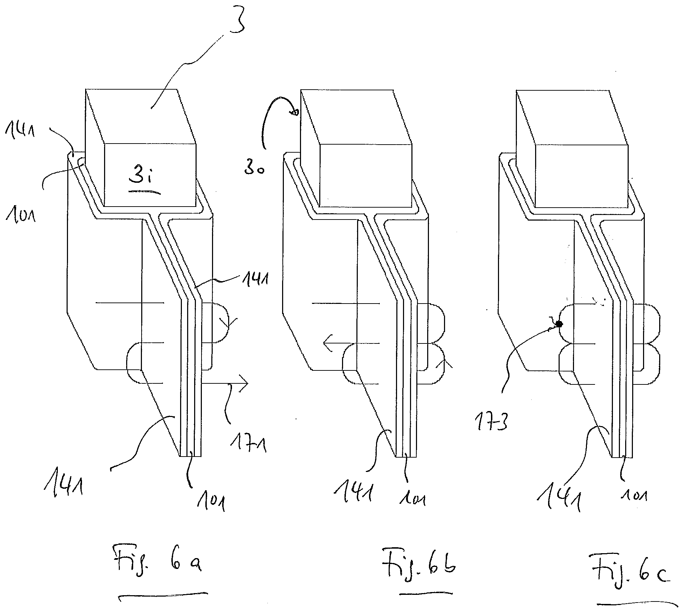

**Fig. 1e** shows three pledges 141, 141' and 141" of the heart valve 100 according to the first exemplary embodiment of the present invention. The pledges 141, 141' and 141" are optional. The benefit of the potential pledges 141, 141' and 141" are discussed with regards to Figs. 6a to 6c. The number of the pledges may correspond to the number of posts 3.

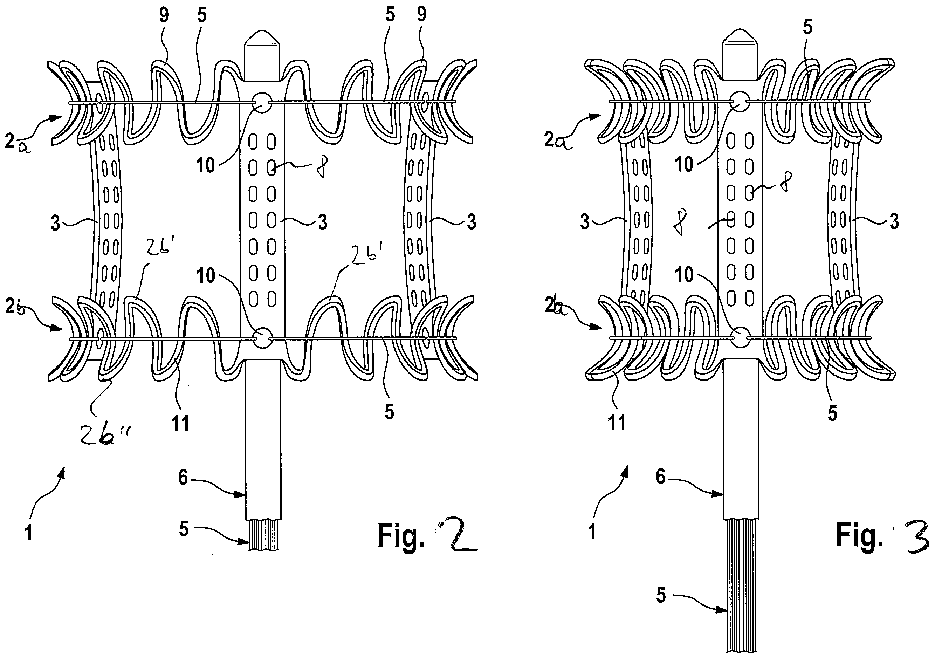

The medical implant according to some embodiments of the present invention comprises a heart valve 100, for example the one discussed with reference to Figs. 1a to 1d or 1e and a frame 1 or supporting structure, for example the one discussed with reference to Figs. 2 and 3. In certain embodiments according to the present invention, the medical implant consists of the heart valve 100 and the frame 1.

**Fig. 2** shows a frame 1 of an exemplary implant according to the present invention. The frame 1 is expandable and can be reduced again in its diameter. The diameter refers to a plane perpendicular to a longitudinal axis of the frame 1. The longitudinal direction also corresponds to the direction of the extension of the catheter 6 shown in Fig. 2.

Frame 1 comprises at least a first or upper - preferably circular - guiding structure or ring 2a and a second or lower - preferably also circular - guiding structure 2b. The guiding structures 2a, 2b are connected to rods or posts 3. In some embodiments, the guiding structures 2a, 2b can - additionally or alternatively or exclusively - fulfill the function of guiding structures for reins 5. The reins 5 form part of a catheter 6 and serve for applying force or tension or stress, respectively, to the guiding structures 2a, 2b for the purpose of expanding or folding the frame 1 in a targeted manner. In the example of Fig. 2, the guiding structures 2a, 2b are each designed having the shape of an outwardly half-open channel through which the reins 5 are guided. The half-open channel is opened in a direction away from the centre of the frame 1. However, the channel can also be shaped to be open to the implant or to any other direction.

In the example of Fig. 2, the guiding structures 2a, 2b are interrupted by posts 3, i.e. the posts 3 are integrated into the guiding structures 2a, 2b such that they form sections of the guiding structures 2a, 2b.

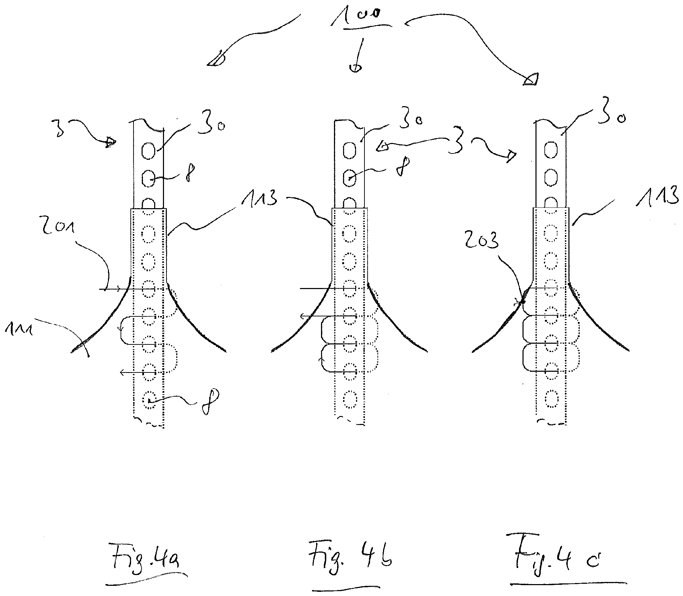

In the embodiment of the frame 1 according to the invention shown in Fig. 2, the posts 3 and/or the guiding structures 2a, 2b have (round or differently shaped, e. g., oval, rectangular, elliptic, and so on) passage means or apertures 10. In the embodiment shown in Fig. 2, they serve as a passage for the reins 5. The posts 3 also have through openings 8, for example eyelets, which can be arranged in two parallel rows as in Fig. 2, in one row as in Figs. 4a to 4c, or in any other arrangement.

Furthermore, the frame 1 can also comprise a number of guiding means 2a, 2b other than two, for example, one, three, four or more guiding means.

The guiding structures 2a, 2b can be arranged circularly, however, they can also be arranged non-circularly.

The guiding structures 2a, 2b can be formed integrally with the implant; however, they can also be fabricated separately.

The guiding structures 2a, 2b can have the shape of a wave or undulation, respectively; however, they can also be fabricated in any other form, in particular, a non-wavy or non-undulating form.

Independent of all other features, frame 1 or parts thereof can be fabricated from flat material, e. g., a material which has been cut with a laser, wherein, e. g., after having designed a pattern in the flat material, the material is reformed into a tube (optionally by connecting, such as welding, longitudinal sides of the former flat material lane or web, respectively). However, frame 1 can also be fabricated from a tubular material directly.

The guiding structures 2a, 2b of frame 1 comprise or consist of a plurality of bars 11 which are each connected to another by means of connecting sections 9. The plurality of bars 11 may be arranged in a zig-zag pattern or an undulating or meandering pattern as is exemplary shown in Fig. 2.

**Fig. 3** shows the frame 1 of Fig. 2. Two reins 5 have been led or guided around the frame 1 and return back to the catheter 6 through the respectively same passage means or apertures 10. The reins 5 apply a tension or stress on the frame 1, and, in consequence, frame 1 is not completely expanded or unfolded. Rather, the diameter of the frame 1 has been reduced or is being hindered from expanding in a free manner.

At least one of the top cuff 121 and the bottom cuff 131 can be secured to the bars 11 of the second or lower guiding structure 2b, for example by using a whip stitch, with, e. g. four stitches per bar 11, preferably evenly spaced. At some or all of the top portions of bars 11, indicated by 2b' in Fig. 2 and Fig. 3, and/or at some of all of the bottom portions of the bars 11, indicated by 2b" in Fig. 2 and Fig. 3, the curved bottom sections 103a, 103a', 103a" of the leaflets 101, 101', 101" are additionally secured to the frame 1, for example once again by means of one or more surgeon's knots. Care should be taken to secured the body 103a, 103a', 103a" only at its rim or seam section.

**Figs. 4a-4c** show how the heart valve 100 of Figs. 1a to 1d or 1e is being fixed or secured to an exemplary post 3 of a frame 1 according to Figs. 2 or 3 in temporal subsequence.

As can be seen in **Fig. 4a**, a suture 201 is pierced through the crown piece 111 and then let through a through hole 8 of the post 3. In doing so, the suture is guided from the outside of the heart valve 100 to the inside of the heart valve 100, leaving a suture tail of at least 2 cm on the outside of the heart valve 100. This tail will later be used to make a knot. Next, a running stitch is created down the post 3 going in and out of the through holes 8 (eyelets) until the suture has been guided through four (preferably neighbouring) through holes 8. The suture is then returned back up the post 3 in and out of the through holes 8 until the through hole 8 below the suture tail is reached, see **Fig. 4b****.** The suture is tied off using the starting suture tail using a surgeon's knot 203 or any other knot or fixture. The knot 203 should be on the outside of the heart valve 100 and/or on an outer side 3o of the post 3 as in **Fig. 4c****.**

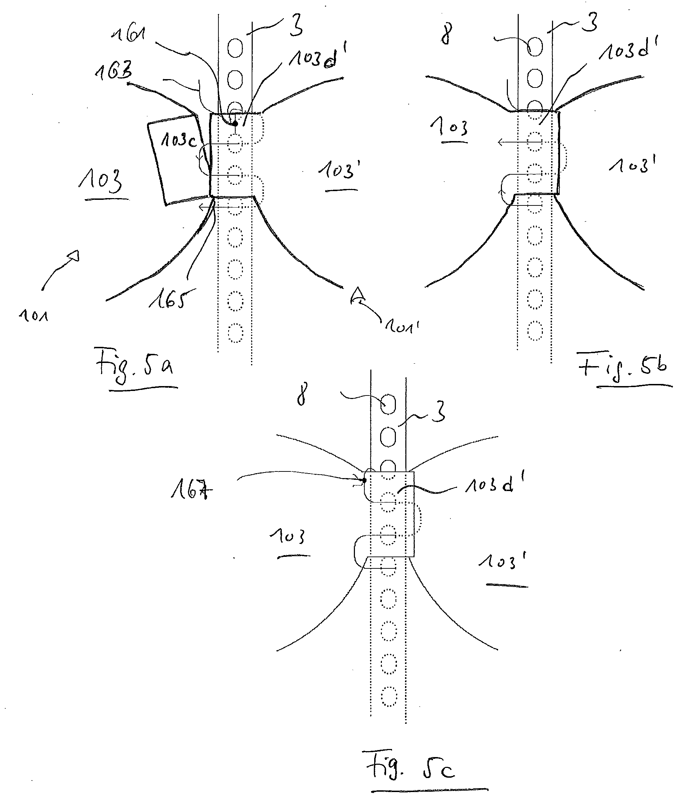

**Figs. 5a** to **5c** show how a tab 103c and a tab 103d' of adjacent leaflets 101 and 101' are attached to a common post 3 of the frame 1.

As can be seen from **Fig. 5a**, tab 103c of leaflet 101 is folded over onto the body 103 of leaflet 101, and a knot 161 is created at the top of tab 103d'. Knot 161 secures the heart valve 100 in place on the frame 1. A suture tail 163 of the suture is not cut. With a running stitch 165 going in and out of preferably each eyelet 8 downwards on the tab 103d' which is placed on an outside of the post 3 the tab 103d' is secured more and more to the post 3 until the bottom of tab 103d' is reached.

Now, as is shown in **Fig. 5b**, tab 103c is folded back over tab 103d' (and hence also on an outside of post 3 although not in contact with the post 3) and the stitch 165 is continued back up the tabs 103c and 103d', again while going in and out of the eyelets 8 until the top of the tabs is reached.

Tail 163 is used for tying off using a suitable knot such as a surgeon's knot 167, see **Fig. 5c****.**

**Figs. 6a** to **6c** show how one of optional pledges 141, 141', 141" as described above is used for reinforcing the connection of the heart valve 100 to the frame 1.

Figs. 6a to 6c show a post 3 having an outside face 3o and an inside face 3i. Outside face 3o is directed to an outside of frame 1 whereas inside face 3i is directed to an inside of frame 1.

A section of post 3 which is enwrapped by a leaflet 101 is now also enwrapped by a pledge 141 such that the free ends of pledge 141 are put above each other or superposed (only separated from each other by the leaflet 101 arranged between the free ends of the pledge 141) in a inner space of frame 1, i. e. at the inside face 3i of post 3.

Starting from the top of pledge 141, a running stitch 171 is run down to the bottom of the pledge (see **Fig. 6a**) and then back up to the top (see **Fig. 6b**). A knot 173 is made using the beginning suture's tail (see **Fig. 6c**). Preferably, the knot 173 is on the side of the pledge 141 and does not contact the leaflet 101.

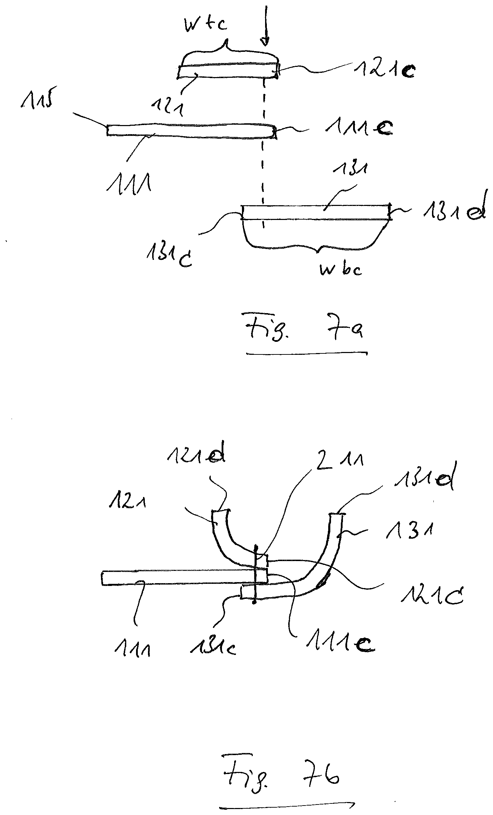

**Figs. 7a** shows the top cuff 121 and the bottom cuff 131 before being interconnected, e. g. sewed, to the crown piece 111, in an exploded view.

If sewed, the arrow indicates the direction of how a first stitch of a suture 211 (see Fig. 7b) may be made.

As can be seen from Fig. 7a, the crown piece 111 may be posed between the top cuff 121 and the bottom cuff 131.

Also, the three elements 111, 121, 131 may be interconnected such that an end of the inner rim or side 121c of the top cuff 121 and an end of the lower rim or side 111c of the crown piece 111 are aligned at one side (see the right hand side in Fig. 7a) such that they end on a common level, whereas an end of the inner rim or side 131c of the bottom cuff 131, which extends remarkably beyond the ends of inner rim 121c and lower side 111c to the right in Fig. 7a, is not aligned with the ends of the lower rim or side 111c and the inner side 121c.

**Fig. 7b** shows the elements of Fig. 7a interconnected with each other, here by means of an exemplary suture, denoted by reference numeral 211. As mentioned above with respect to certain exemplary embodiments according to the present invention suture 211 may run along the entire length 1 (see Figs. 1b to 1d). Suture 211 may be the sole or only suture used for interconnecting these three elements.

Both the top cuff 121 and the bottom cuff 131 are bend upwards (with regards to Fig. 7b) to assume a c-shape which is open towards the top of Fig. 7b. The c-shape is very similar to the shape bars 11 assume in Fig. 2. Hence, the c-shape is well-suited if the combination of top cuff 121 and bottom cuff 131 is to cover bars 11 in practise from in inner side of the guiding structure 2a, 2b shown in Fig. 2.

The expansion of frame 1 may benefit in the present exemplary embodiment from the internal stress or from shape-memory capacities of frame 1. The frame 1 may be manufactured from Nitinol or comprise such material.

### Reference numerals

- 1: frame
- 2a: first or upper guiding structure
- 2b: second or lower guiding structure
- 2b': top portion of the bars
- 2b": bottom portion of the bars
- 3: posts
- 3i: inside face of post 3
- 3o: outside face of post 3
- 5: reins or tension thread(s)
- 6: catheter
- 8: through opening(s) or, as in particular embodiments; eyelet(s)
- 9: connecting sections
- 10: apertures
- 11: bars
- 100: heart valve
- 101, 101', 101": leaflets
- 103: body of the heart valve 100
- 103a: curved bottom section
- 103b: rim section of the body of the leaflet
- 103c, 103d: opposing tabs
- 111: crown piece
- 111a, 111b: free end
- 111c: lower rim or side of the crown piece
- 111e: end of the lower rim or side of the crown piece
- 113, 113', 113": small tabs
- 115, 115' 115": round portions

- 121: top cuff
- 121a, 121b: ends of top cuff
- 121c: inner rim or side of top cuff
- 121d: outer rim or side of top cuff
- 121e: end of the inner rim or side of top cuff
- 131: bottom cuff
- 131a, 131b: ends of bottom cuff
- 131c: inner rim or side of bottom cuff
- 131d: outer rim or side of bottom cuff
- 131e: end of the inner rim or side of bottom cuff

- 141, 141', 141": pledges

- 161: knot
- 163: suture tail
- 165: running stitch or suture
- 167: knot

- 173: knot
- 201: suture between crown piece and through hole
- 203: knot
- 211: suture interconnecting both the top cuff and the bottom cuff to the crown piece
- l: length of the stripe
- rtc: radius
- rbc: radius
- wtc: width of top cuff
- wbc: width of bottom cuff

## Claims

1. A foldable and/or unfoldable medical implant comprising a heart valve (100) and a foldable and/or unfoldable frame (1) being interconnected to the heart valve (100), by sewing or sewing alone, the frame (1) comprising:
- at least a first guiding structure (2a) for guiding at least one tension thread (5) for folding and/or unfolding the frame (1) around or along the Frame (1), preferably at an outside or an outer circumference thereof;
- at least a second guiding structure (2b), different from the first guiding structure (2a) for guiding at least one tension thread (5) for folding and/or unfolding the frame (1) around or along the frame (1), preferably at an outside or an outer circumference thereof;
- at least two, preferably three, posts (3), the posts (3) being arranged between the first and the second guiding structure (2a, 2b) in order to interconnect the first and the second guiding structure (2a, 2b) with each other and/or to maintain the distance between them;
and the heart valve (100), comprising:
- at least two leaflets (101, 101', 101");
**characterized in that** the heart valve (100) also comprises:
- at least one crown piece (111) interconnected to the leaflets (101, 101', 101"), the crown piece (111) preferably intended to be interconnected to a frame (1) of the medical implant;
- a top cuff (121); and
- a bottom cuff (131),
the crown piece (111), the top cuff (121) and the bottom cuff (131) each being ring-shaped, and each of the top cuff (121) and the bottom cuff (131) being interconnected with the crown piece (111),
wherein
the crown piece (111) is sewed to the posts (3).

2. The implant according to claim 1, wherein at least one of the ring-shaped top cuff (121) and the ring-shaped bottom cuff (131) is formed from a stripe or comprises a stripe, wherein the stripe is curved along its length.

3. The implant according to claim 1 or 2, wherein the top cuff (121) has a width (wtc) that is smaller than the width (wbc) of the bottom cuff (131).

4. The implant according to any one of the preceding claims, wherein all leaflets (101, 101', 101") are sewed to the crown piece (111) by means of one common suture or one suture each.

5. The implant according to any one of the preceding claims, wherein both the top cuff (121) and the bottom cuff (131) are sewed to the crown piece (111) by one suture.

6. The implant according to any one of the preceding claims, wherein the crown piece (111) or a section thereof is interposed between the top cuff (121) and the bottom cuff (131).

7. The medical implant according any one of the preceding claims, wherein the crown piece (111) is sewed to the posts (3) via through holes (8) of the posts (3), preferably through at least three or four, preferably consecutive through holes (8), preferably by means of tabs (113, 113', 113") of the crown piece (111) or by leaving out the tabs (113, 113', 113"), preferably by one suture (201).

8. The medical implant according to claim 7, wherein the suture (201) was started from an outer side (3o) of the posts (3) to an inner side (3i) of the posts (3), preferably such that the only knot (203) that interconnects both ends of the suture (201) is arranged on the outer side (3o) of the posts (3).

9. The medical implant according to any one of the preceding claims, wherein the posts (3) are arranged inside a circle or an area circumscribed by the crown piece (111).

10. The medical implant according to any one of the preceding claims, wherein the posts (3) are arranged outside the circle or the area circumscribed by at least one of the top cuff (121) and the bottom cuff (131).

11. The medical implant according to any one of the preceding claims, wherein the at least one of the first and the second guiding structure (2a, 2b) comprises or consists of bars (11) that are interconnected to each other, preferably so as to form a zig-zag pattern or an undulating or meandering pattern, and wherein the bars (11) are arranged outside the circle or the area circumscribed by at least one of the top cuff (121) and the bottom cuff (131).

12. The medical implant according to any one of the preceding claims, wherein the leaflets (101, 101', 101") have a first tab (103c, 103c', 103c") and a second tab (103d, 103d', 103d") arranged at opposite ends of the leaflets (101, 101', 101"), and wherein tabs (103c, 103c', 103c", 103d, 103d', 103d") are sewed onto the posts (3) of the frame (1).

13. The medical implant according to the preceding one wherein tabs (103c, 103c', 103c", 103d, 103d', 103d") of two adjacent leaflets (101, 101', 101") are sewed to one post (3) in an overlapping manner.

14. The medical implant according to claim 12 or 13, wherein the tab (103d') of a first leaflet (103') is sewed onto a post (3) first, and wherein the tab (103c) of a second leaflet (103) is sewed onto both the tab (103d') of the first leaflet (103') and the post (3) in one running stitch or with one suture (165).

15. A medical implant according to any one of the preceding claims, wherein the implant is a stent or a cardiac or heart valve assembly.

## Patentansprüche

1. Faltbares und/oder entfaltbares medizinisches Implantat mit einer Herzklappe (100) und einem faltbaren und/oder entfaltbaren Rahmen (1) welcher mit der Herzklappe (100) mittels Nähen, oder ausschließlich mittels Nähen, verbunden ist, wobei der Rahmen (1) umfasst:
- wenigstens eine erste Führungsstruktur (2a) zum Führen wenigstens eines Spannfadens (5) zum Falten und/oder Entfalten des Rahmens (1) um oder entlang des Rahmens (1), vorzugsweise an einer Außenseite oder an einer äußeren Peripherie desselben;
- wenigstens eine, sich von der ersten Führungsstruktur (2a) unterscheidende, zweite Führungsstruktur (2b) zum Führen wenigstens eines Spannfadens (5) zum Falten und/oder Entfalten des Rahmens (1) um oder entlang des Rahmens (1), vorzugsweise an einer Außenseite oder an einer äußeren Peripherie desselben;
- wenigstens zwei, vorzugsweise drei Säulen (3), wobei die Säulen (3) derart zwischen der ersten und der zweiten Führungsstruktur (2a, 2b) angeordnet sind, dass sie die erste und die zweite Führungsstruktur (2a, 2b) miteinander verbinden und/oder dass sie einen Abstand zwischen ihnen erhalten;
wobei die Herzklappe (100) ferner umfasst:
- wenigstens zwei Klappensegel (101, 101', 101");
**dadurch gekennzeichnet, dass** die Herzklappe (100) ferner umfasst:
- wenigstens ein Kronenstück (111), welches mit den Klappensegeln (101, 101', 101") verbunden ist, wobei das Kronenstück (111) vorzugsweise dafür vorgesehen ist, mit einem Rahmen (1) des medizinischen Implantats verbunden zu werden;
- eine obere Manschette (121); und
- eine untere Manschette (131),
wobei das Kronenstück (111), die obere Manschette (121) und die untere Manschette (131) jeweils ringförmig ausgebildet sind und wobei die obere Manschette (121) und die untere Manschette (131) jeweils mit dem Kronenstück (111) verbunden sind;
wobei das Kronenstück (111) mit den Säulen (3) vernäht ist.

2. Implantat nach Anspruch 1, wobei wenigstens die ringförmige obere Manschette (121) und/oder wenigstens die ringförmige untere Manschette (131) aus einem Streifen ausgebildet ist, oder einen Streifen umfasst, wobei der Streifen entlang seiner Länge gekrümmt ist.

3. Implantat nach Anspruch 1 oder 2, wobei die obere Manschette (121) eine Breite (wtc) hat, welche kleiner als die Breite (wbc) der unteren Manschette (131) ist.

4. Implantat nach einem der vorangegangenen Ansprüche, wobei alle Klappensegel (101, 101', 101") mittels einer gemeinsamen Naht oder mittels jeweils einer einzigen Naht mit dem Kronenstück (111) vernäht sind.

5. Implantat nach einem der vorangegangenen Ansprüche, wobei die obere Manschette (121) und die untere Manschette (131) beide mittels einer Naht mit dem Kronenstück (111) vernäht sind.

6. Implantat nach einem der vorangegangenen Ansprüche, wobei das Kronenstück (111), oder ein Abschnitt hiervon, zwischen der oberen Manschette (121) und der unteren Manschette (131) vorliegt.

7. Implantat nach einem der vorangegangenen Ansprüche, wobei das Kronenstück (111) mit den Säulen (3) durch Durchgangsöffnungen (8) der Säulen (3) vernäht ist, vorzugsweise durch drei oder vier, vorzugsweise aufeinander folgenden Durchgangsöffnungen (8), vorzugsweise mittels Zungen (113, 113', 113") des Kronenstücks (111) oder beim Auslassen der Zungen (113, 113', 113"), vorzugsweise mittels einer einzigen Naht (201).

8. Implantat nach Anspruch 7, wobei die Naht (201) von einer Außenseite (3o) der Säulen (3) hin zu einer Innenseite (3i) der Säulen (3) beginnt, vorzugsweise derart, dass der einzige Knoten (203), welcher beide Enden der Naht (201) miteinander verbindet, auf der Außenseite (3o) der Säulen (3) angeordnet ist.

9. Implantat nach einem der vorangegangenen Ansprüche, wobei die Säulen (3) innerhalb eines Kreises oder einer Fläche angeordnet sind, welche vom Kronenstück (111) begrenzt ist.

10. Implantat nach einem der vorangegangenen Ansprüche, wobei die Säulen (3) außerhalb eines Kreises oder einer Fläche angeordnet sind, welche wenigstens von der unteren Manschette (121) und/oder von der unteren Manschette (131) begrenzt ist.

11. Implantat nach einem der vorangegangenen Ansprüche, wobei die erste und/oder die zweite Führungsstruktur (2a, 2b) Balken (11) umfassen, oder hieraus bestehen, welche derart miteinander verbunden sind, dass sie ein Zig-Zag-Muster oder ein welliges oder gewundenes Muster bilden, und wobei die Balken (11) außerhalb eines Kreises oder einer Fläche angeordnet sind, welche wenigstens von der unteren Manschette (121) und/oder von der unteren Manschette (131) begrenzt ist.

12. Implantat nach einem der vorangegangenen Ansprüche, wobei die Klappensegel (101, 101', 101") eine erste Zunge (103c, 103c', 103c") und eine zweite Zunge (103d, 103d', 103d") aufweisen, welche an gegenüberliegenden Enden der Klappensegel (101, 101', 101") angeordnet sind, und wobei die Zungen (103c, 103c', 103c", 103d, 103d', 103d") mit den Säulen (3) des Rahmens (1) vernäht sind.

13. Implantat nach einem der vorangegangenen Ansprüche, wobei die Zungen (103c, 103c', 103c", 103d, 103d', 103d") zweier benachbarter Klappensegel (101, 101', 101") mit einer Säule (3) überlappend vernäht sind.

14. Implantat nach Anspruch 12 oder 13, wobei die Zunge (103d') eines ersten Klappensegels (103') zunächst mit einer Säule (3) vernäht wird, und wobei die Zunge (103c) eines zweiten Klappensegels (103) jeweils mit der Zunge (103d') des ersten Klappensegels (103') und der Säule (3) mittels eines Steppstichs oder mittels einer einzigen Naht (165) vernäht ist.

15. Implantat nach einem der vorangegangenen Ansprüche, wobei das Implantat ein Stent oder eine Herz- oder Herzklappenanordnung ist.

## Revendications

1. Un implant médical pliable et/ou dépliable comprenant une valvule cardiaque (100) et un support (1) pliable et/ou dépliable interconnecté avec la valvule cardiaque (100) au moyen d'une suture ou uniquement par suture, le support (1) comprenant :
- au moins une première structure de guidage (2a) pour guider au moins un fil de tension (5) prévu pour plier et/ou déplier le support (1) autour ou le long du support (1), de préférence à l'extérieur ou sur une périphérie extérieure de celui-ci ;
- au moins une seconde structure de guidage (2b) différente de la première structure de guidage (2a) pour guider au moins un fil de tension (5) prévu pour plier et/ou déplier le support (1) autour ou le long du support (1), de préférence à l'extérieur ou sur une périphérie extérieure de celui-ci ;
- au moins deux, préférablement trois montants (3) étant agencés entre la première et la second structure de guidage (2a, 2b) de façon à interconnecter la première et la seconde structure de guidage (2a, 2b) l'une avec l'autre et/ou de façon à maintenir une distance entre celles-ci ;
la valvule cardiaque (100) comprenant en outre :
- au moins deux feuillets (101, 101', 101") ;
**caractérisé en ce que** la valvule cardiaque (100) comprend de plus :
- au moins une pièce en forme de couronne (111) interconnectée avec les feuillets (101, 101', 101"), la pièce en forme de couronne (111) étant préférablement prévue pour être interconnectée à un support (1) de l'implant médical ;
- une manchette supérieure (121) ; et
- une manchette inférieure (131),
la pièce en forme de couronne (111), la manchette supérieure (121) et la manchette inférieure (131) étant, chacune, de forme annulaire, et la manchette supérieure (121) ainsi que la manchette inférieure (131) étant, chacune, interconnectée avec la pièce en forme de couronne (111),
la pièce en forme de couronne (111) étant suturée sur les montants (3).

2. L'implant selon la première revendication, où au moins la manchette supérieure annulaire (121) ou la manchette inférieure annulaire (131), est formée d'une bande ou comprend une bande, la bande étant incurvée sur sa longueur.

3. L'implant selon la première ou la seconde revendication, où la manchette supérieure (121) a une largeur (wtc) inférieure à la largeur (wbc) de la manchette inférieure (131)

4. L'implant selon l'une quelconque des revendications précédentes, où tous les feuillets (101, 101', 101") sont suturés sur la pièce en forme de couronne (111) au moyen d'une suture commune ou chaque feuillet au moyen d'une suture individuelle.

5. L'implant selon l'une quelconque des revendications précédentes, où la manchette supérieure (121) et la manchette inférieure (131) sont suturées à la pièce en forme de couronne (111) au moyen d'une suture.

6. L'implant selon l'une quelconque des revendications précédentes, où la pièce en forme de couronne (111), ou une section de celle-ci, est interposée entre la manchette supérieure (121) et la manchette inférieure (131).

7. L'implant médical selon l'une quelconque des revendications précédentes, où la pièce en forme de couronne (111) est suturée sur les montants (3) au moyen d'orifices (8) des montants (3), de préférence au moyen de trois ou quatre orifices (8), préférablement consécutifs, de préférence au moyen d'onglets (113, 113', 113") de la pièce en forme de couronne (111) ou en omettant les onglets (113, 113' , 113"), préférablement au moyen d'une suture (201).

8. L'implant médical selon la revendication 7, où la suture (201) débute d'un côté extérieur (3o) des montants (3) vers un côté intérieur (3i) des montants (3), préférablement de sorte à ce que l'unique noeud (203) interconnectant les deux extrémités de la suture (201) soit situé sur le côté extérieur (3o) des montants (3).

9. L'implant médical selon l'une quelconque des revendications précédentes, où les montants (3) sont agencés à l'intérieur d'un cercle ou d'une surface circonscrite par la pièce en forme de couronne (111).

10. L'implant médical selon l'une quelconque des revendications précédentes, où les montants (3) sont agencés à l'extérieur du cercle ou de la surface circonscrite tout au moins par la manchette supérieure (121) ou la manchette inférieure (131).

11. L'implant médical selon l'une quelconque des revendications précédentes, où tout au moins la première structure de guidage ou la seconde structure de guidage (2a, 2b) comprend ou consiste de tiges (11) interconnectées les unes avec les autres, de façon à préférablement former un motif en zigzag, ondulé ou en méandre, dans lequel les tiges (11) sont agencées à l'extérieur du cercle ou de la surface circonscrite tout au moins par la manchette supérieure (121) ou la manchette inférieure (131).

12. L'implant médical selon l'une quelconque des revendications précédentes, où les feuillets (101, 101', 101") ont un premier onglet (103c, 103c', 103c") et un second onglet (103d, 103d', 103d") agencés sur des extrémité opposées des feuillets (101, 101', 101") et où les onglets (103c, 103c', 103c", 103d, 103d', 103d") sont suturés sur les montants (3) du support (1).

13. L'implant médical selon l'une quelconque des revendications précédentes, où les onglets (103c, 103c' , 103c", 103d, 103d', 103d") de deux feuillets adjacents (101, 101', 101") sont suturés sur un montant (3) de manière chevauchante.

14. L'implant médical selon la revendication 12 ou 13, où l'onglet (103d') d'un premier feuillet (103') est tout d'abord suturé sur un montant (3) et où l'onglet (103c) d'un second feuillet (103) est suturé sur l'onglet (103d') du premier feuillet (103') ainsi que sur le montant (3) au moyen d'un point piqué courant ou au moyen d'une seule suture (165).

15. L'implant médical selon l'une quelconque des revendications précédentes, où l'implant est un stent, un arrangement cardiaque ou de valvule cardiaque.
